# EUROPÄISCHE PATENTSCHRIFT

(11) **EP 1 458 670 B1**
(45) Veröffentlichungstag und Bekanntmachung des Hinweises auf die Patenterteilung: **09.03.2011**
(21) Anmeldenummer: 02798321.2
(22) Anmeldetag: 04.12.2002
(51) Int. Cl.: C07C 227/16, C07D 231/14

(54) **VERFAHREN ZUR HERSTELLUNG VON 2-HALOGENACYL-3-AMINO-ACRYLSÄURE- DERIVATEN**
METHOD FOR PRODUCING 2-HALOGENACYL-3-AMINO-ACRYLIC ACID DERIVATIVES
PROCEDE DE PRODUCTION DE DERIVES D'ACIDE 2-HALOGENACYL-2-AMINO-ACRYLIQUE

(30) Priorität: 17.12.2001 DE 10161978
(43) Veröffentlichungstag der Anmeldung: 22.09.2004
(73) Patentinhaber: Saltigo GmbH, 40764 Langenfeld (DE)
(72) Erfinder: LUI, Norbert, 51061 Köln (DE); BRACKEMEYER, Thomas, D-50679 Köln (DE); MÜLLER, Peter, 51519 Odenthal (DE); SCHNEIDER, Marielouise, 51373 Leverkusen (DE)
(74) Vertreter: Wichmann, Birgid
(86) Internationale Anmeldenummer: PCT/EP2002/013721
(87) Internationale Veröffentlichungsnummer: WO 2003/051820

(56) Entgegenhaltungen:
- EP-A- 0 285 947
- EP-A- 1 000 926
- EP-A- 1 132 375
- BARTNIK R ET AL: "A New Synthesis of Enaminoketones" TETRAHEDRON LETTERS, ELSEVIER SCIENCE PUBLISHERS, AMSTERDAM, NL, Bd. 37, Nr. 48, 25. November 1996 (1996-11-25), Seiten 8751-8754, XP004068768 ISSN: 0040-4039 in der Anmeldung erwähnt
- JAMES R BECK ET AL: "Synthesis of 1-(1,1-Dimethyletyl)1H-pyrazole-4- carboxylate Ester Derivatives" JOURNAL OF HETEROCYCLIC CHEMISTRY, HETEROCORPORATION. PROVO, US, Bd. 24, Nr. 3, Mai 1987 (1987-05), Seiten 693-695, XP002126048 ISSN: 0022-152X

## Beschreibung

Die vorliegende Erfindung betrifft ein verbessertes Verfahren zur Herstellung von 2-Halogenacyl-3-amino-acrylsäure-derivaten und daraus erhältlichen Pyrazol-4-carbonsäure-derivaten.

2-Halogenacyl-3-amino-acrylsäure-derivate wie z.B. 2-Trifluoracetyl-3-amino-acrylsäureester sind wertvolle Zwischenprodukte bei der Herstellung von substituierten Pyrazolen, die als Fungizide, Pestizide und Herbizide Verwendung finden.

Aus EP-A 1 000 926 ist ein Verfahren zur Herstellung von 2-Trihalogenacetyl-3-amino-acrylsäureestern bekannt, bei der Trihalogenacetylacetate mit orthoAmeisensäure-derivaten substituiert werden. Die erreichten Ausbeuten der Reaktion liegen jedoch bei lediglich 61,8 % und sind für eine industrielle Anwendung nicht akzeptabel.

Eine weitere Methode zur Herstellung derartiger Verbindungen ist von Bartnik et. al. (Tetrahedron Lett. (1996), 37(48), 8751-8754) beschrieben. Dabei werden β-Chloracroleine mit sek. Aminen in Diethylether bei RT und Ausbeuten von 44 bis 84 % umgesetzt.

Nachteilig an diesem Verfahren ist jedoch, dass die als Ausgangsverbindungen eingesetzten Chloracroleine schwierig herzustellen und daher für eine technische Anwendung zu teuer sind.

Es bestand daher das Bedürfnis ausgehend von einfach zugänglichen Edukten ein verbessertes Verfahren zur Herstellung von 2-Halogenacyl-3-amino-acrylsäure-derivaten zu entwickeln.

Es wurde nun ein Verfahren zur Herstellung von 2-Halogenacyl-3-amino-acrylsäureestern gefunden, das dadurch gekennzeichnet ist, dass
a) N-substituierte 3-Amino-acrylsäureester mit Halogenalkylcarbonsäureanhydriden, wobei unter Halogenalkylcarbonsäureanhydride auch Halogenalkylcarbonsäurechloride als die gemischten Anhydride von Halogenalkylcarbonsäuren mit Halogenwasserstoffsäuren verstanden werden, in Gegenwart von Base und gegebenenfalls in Gegenwart von Lösungsmittel umgesetzt werden. Gegebenenfalls können die auf diese Weise erhaltenen 2-Halogenacyl-3-amino-acrylsäure-ester
b) durch Umsetzung mit Hydrazinen in 3-Halogenalkyl-4-pyrazolcarbonsäure-ester überführt werden, die
c) gegebenenfalls durch saure oder alkalische Verseifung weiter zu 3-Halogenalkyl-4-pyrazolcarbonsäuren umgesetzt werden können.

3-Amino-acrylsäureester sind im Rahmen der Erfindung beispielsweise und bevorzugt solche der allgemeinen Formel (I), 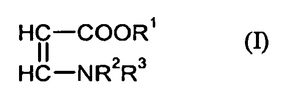 in der
- R¹: für C₁-C₁₂-Alkyl, C₆-C₁₈-Aryl oder C₇-C₁₉-Arylalkyl stehen und
- R² und R³: jeweils unabhängig voneinander für C₁-C₁₂-Alkyl oder C₇-C₁₉-Arylalkyl stehen.

Bevorzugt steht R¹ für C₁-C₄-Alkyl, besonders bevorzugt für Methyl oder Ethyl und R² und R³ jeweils unabhängig voneinander bevorzugt für C₁-C₄-Alkyl, besonders bevorzugt für Methyl oder Ethyl.

Besonders bevorzugte 3-Amino-acrylsäureester der allgemeinen Formel (I) sind 3-(N,N-Dimethylamino)-acrylsäuremethylester und 3-(N,N-Diethylamino)-acrylsäure-ethylester von denen sind 3-(N,N-Dimethylamino)-acrylsäuremethylester noch weiter bevorzugt ist.

Die einzusetzenden 3-Amino-acrylsäureester sind nach Literatur oder analog dazu herstellbar (EP-A 608 725).

Alkyl steht im Rahmen der Erfindung einen geradkettigen, cyclischen, verzweigten oder unverzweigten Alkyl-Rest, der gegebenenfalls durch C₁-C₆-Alkoxy-Reste wie zum Beispiel Methoxy oder Ethoxy weiter substituiert sein kann. Gleiches gilt für den Alkylenteil eines Arylalkyl-Restes.

Beispielsweise steht C₁-C₄-Alkyl für Methyl, Ethyl, Ethoxyethyl, n-Propyl, Isopropyl, n-Butyl und tert.Butyl, C₁-C₈-Alkyl; darüber hinaus für n-Pentyl, Cyclohexyl, n-Hexyl, n-Octyl oder iso-Octyl, C₁-C₁₂-Alkyl darüber hinaus z.B. für n-Decyl und n-Dodecyl. Alkoxy steht im Rahmen der Erfindung einen geradkettigen, cyclischen, verzweigten oder unverzweigten Alkoxy-Rest, der gegebenenfalls durch C₁-C₆-Alkoxy-Reste wie zum Beispiel Methoxy oder Ethoxy weiter substituiert sein kann.

Beispielsweise steht C₁-C₆-Alkoxy für Methoxy, Ethoxy, 2-ethoxy-ethoxy, n-Propoxy, Isopropoxy, n-Butoxy, tert.Butoxy oder Cyclohexyloxy.

Aryl steht im Rahmen der Erfindung beispielsweise und bevorzugt für carbocyclische aromatische Reste mit 6 bis 18 Gerüstkohlenstoffatomen (C₆-C₁₈-Aryl), wie z.B. Phenyl oder Naphthyl.

Weiterhin können die carbocyclischen aromatischen Reste mit bis zu fünf gleichen oder verschiedenen Substituenten pro Cyclus substituiert sein, wie beispielsweise ausgewählt aus der Gruppe Chlor, Fluor, Nitro, Cyano, C₁-C₄-Alkyl wie z.B. Methyl oder Ethyl, C₁-C₄-Acyl wie zum Beispiel Acetyl, C₁-C₄-Alkoxy wie z.B. Methoxy oder Ethoxy, C₆-C₁₂-Aryl wie z.B. Phenyl, C₇-C₁₃-Arylalkyl wie z.B. Benzyl, C₆-C₁₂-Aryloxy wie z.B. Phenoxy.
Beispiele für C₆-C₁₀-Aryl-Reste sind Phenyl, o-,m-, p-Tolyl, o-, m-, p-Anisyl und Naphthyl für C₆-C₁₈-Aryl darüberhinaus z.B.Anthracenyl.

Gleiches gilt für den Arylteil eines Arylalkyl-Restes. C₇-C₁₃-Arylalkyl steht beispielsweise für Benzyl oder die isomeren 1-Methylbenzyle, C₇-C₁₃-Arylalkyl darüberhinaus z.B. für Fluorenyl.

Im Schritt a) des erfindungsgemäßen Verfahrens werden Halogenalkylcarbonsäureanhydride eingesetzt.

Unter Halogenalkylcarbonsäureanhydriden sind im Rahmen der Erfindung nicht nur symmetrische Anhydride oder gemischte Anhydride von verschiedenen Halogenalkylcarbonsäuren zu verstehen, sondern auch gemischte Anhydride von Halogenalkylcarbonsäuren mit organischen Säuren wie zum Beispiel Sulfonsäuren oder anorganischen Säuren wie zum Beispiel Halogenwasserstoffsäuren. Letztere werden auch als Halogenalkylcarbonsäurehalogenide bezeichnet.

Als Halogenalkylcarbonsäureanhydride werden beispielsweise und bevorzugt solche der allgemeinen Formeln (IIa) eingesetzt, 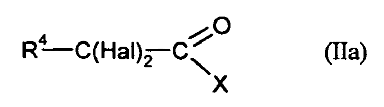 in der
- X: für Chlor, Brom oder Iod, bevorzugt Chlor und
- Hal: jeweils unabhängig voneinander für Chlor oder Fluor, bevorzugt Fluor und
- R⁴: für Chlor, Fluor C₁-C₁₂-Halogenalkyl, C₁-C₁₂-Alkyl, C₆-C₁₈-Aryl oder C₆- C₁₉-Arylalkyl, bevorzugt Chlor, Fluor, Trifluormethyl, Pentafluorethyl, Nona- fluorbutyl und C₁-C₄-Alkyl, besonders bevorzugt für Fluor steht.

Weiterhin werden als Halogenalkylcarbonsäureanhydride beispielsweise und bevorzugt solche der allgemeinen Formel (IIb) eingesetzt 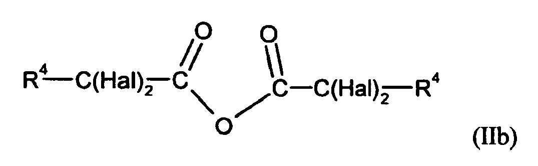 in der

Hal und die Reste R⁴ jeweils unabhängig voneinander die gleiche Bedeutung und Vorzugsbereiche besitzen, die unter der allgemeinen Formel (IIa) angegeben wurden. Bevorzugt sind in der allgemeinen Formel (IIb) die Reste R⁴ identisch.

Halogenalkyl steht im Rahmen der Erfindung beispielsweise und bevorzugt für einen verzweigten oder unverzweigten, offenkettigen oder cyclischen Alkylrest, der einfach, mehrfach oder vollständig durch Halogenatome ausgewählt aus der Gruppe Chlor und Fluor substituiert ist.

Beispielsweise und bevorzugt steht C₁-C₁₂-Halogenalkyl für Trifluormethyl, Trichlormethyl, 2,2,2-Trifluorethyl, Pentafluorethyl und Nonafluorbutyl.

Beispielsweise und bevorzugt werden als Halogenalkylcarbonsäureanhydride Trifluoressigsäureanhydrid, Trifluoressigsäurechlorid, Trichloressigsäureanhydrid und Trichloressigsäurechlorid eingesetzt.

Das molare Verhältnis von Halogenalkylcarbonsäureanhydriden zu eingesetzten 3-Amino-acrylsäureestern kann beispielsweise 0,3 bis 1,5 betragen, bevorzugt 0,8 bis 1,1, besonders bevorzugt 0,95 bis 1,05.

Als Basen eignen sich beispielsweise und bevorzugt tertiäre Stickstoffbasen, Carbonate, Hydride.

Besonders bevorzugt werden tertiäre Stickstoffbasen, wie zum Beispiel tertiäre Amine, substituierte oder unsubstituierte Pyridine und substituierte oder unsubstituierte Chinoline, eingesetzt.

Ganz besonders bevorzugt werden als Basen Pyridin, 2-, 3-, 4-Picolin, 2,6-Lutidin, Chinolin und solche der allgemeinen Formel (IIIa) eingesetzt,

NR⁵R⁶R⁷ (IIIa)

in der R⁵, R⁶ und R⁷ jeweils unabhängig voneinander für C₁-C₁₆-Alkyl, C₇-C₁₉-Arylalkyl oder C₆-C₁₈-Aryl stehen oder jeweils zwei Reste zusammen auch Teil eines 5 bis 8 gliedrigen N-heterocyclischen Restes sein können, oder alle drei Reste zusammen Teil eines N-heterobicyclischen oder N-heterotricyclischen Restes mit 5 bis 9 Ringatomen pro Cyclus sein können die gegebenenfalls auch andere Heteroatome wie zum Beispiel Sauerstoff enthalten können.

Ebenfalls bevorzugt können als Basen solche der allgemeinen Formel (IIIb) eingesetzt werden,

R⁸R⁹-N-A-NR¹⁰R¹¹ (IIIb),

in der
- A: für C₂-C₈-Alkylen wie beispielsweise und bevorzugt 1,2-Ethylen, 1,3 Propylen, 2,3-Butylen, 1,2-cyclohexylen oder C₆-C₁₈-Arylen wie zum Bei- spiel 1,2-Phenylen stehen kann und
die Reste R⁸, R⁹, R¹⁰ und R¹¹ jeweils unabhängig voneinander für C₁-C₁₈-Alkyl, C₇-C₁₉-Arylalkyl oder C₆-C₁₈-Aryl stehen oder jeweils zwei Reste zusammen auch Teil eines 5 bis 8 gliedrigen N-heterocyclischen Ringes oder zwischen den beiden Stickstoffatomen verbrücken sein können oder alle vier Reste zusammen Teil eines Bis-N-heterobicyclischen oder Bis-N-heterotricyclischen Restes mit 5 bis 9 Ringatomen pro Cyclus sein können die gegebenenfalls auch andere Heteroatome wie zum Beispiel Sauerstoff enthalten können.

Bevorzugte Beispiele für Basen der allgemeinen Formel (IIIa) sind Trimethylamin, Triethylamin, Tri-n-propylamin, Tri-n-butylamin, Tri-n-hexylamin, Tri-cyclohexylamin, N-Methyl-cyclohexylamin, N-Ethyl-cyclohexylamin, N-Methylpyrrolidin, N-Ethylpyrrolidin, N-Methylpiperidin, N-Ethylpiperidin, N,N-Dimethylanilin, N-Methylmorpholin, N-Ethylmorpholin, Dimethylhexadecylamin und N,N-Dimethylbenzylamin.

Gleichsam bevorzugte Beispiele für Basen der allgemeinen Formel (IIIb) sind N,N,N,N-Tetramethylethylendiamin, N,N-Dimethyl-1,4-diazacyclohexan, N,N-Diethyl-1,4-diazacyclohexan, 1,8-Bis-(Dimethylamino)naphthalin, Diazabicyclooctan (DABCO), Diazabicyclononan (DBN) und Diazabicycloundecan (DBU).

Ganz besonders bevorzugt wird als Base Triethylamin eingesetzt.

Das molare Verhältnis von Base zu eingesetzten Halogenalkylcarbonsäureanhydriden kann beispielsweise 0,3 bis 3 betragen, bevorzugt 1,0 bis 2,0, besonders bevorzugt 1,05 bis 1,5.

Der Einsatz größerer Mengen an Base ist unkritisch aber unwirtschaftlich.

Die Umsetzung der 3-Amino-acrylsäureester mit Halogenalkylcarbonsäureanhydriden in Gegenwart von Base kann beispielsweise bei Temperaturen von -30 bis 120°C, bevorzugt -10 bis 20°C durchgeführt werden.

Bevorzugt wird die Reaktion in Gegenwart von Lösungsmittel durchgeführt.

Geeignete Lösungsmittel sind beispielsweise aliphatische oder aromatische Kohlenwasserstoffe, die weiterhin durch Fluor- und Chloratome substituiert sein können, Ether, wie z.B. THF oder Dioxan.

Beispielsweise und bevorzugt seien genannt Toluol, o-,m-,p-Xylol, Chlorbenzol, Fluorbenzol, die isomeren Chlor-Fluorbenzole, Dichlormethan, n-Hexan, Cyclohexan, Methylcyclohexan, Heptan, Octan, iso-Octan, Petrolether, Benzinfraktionen, THF oder Dioxan, besonders bevorzugt ist Toluol.

Pro Mol 3-Amino-acrylsäurederivat kann man beispielsweise 50 bis 1000ml Lösungsmittel einsetzen. Bevorzugt liegt diese Menge bei 100 bis 600 ml. Größere Lösungsmittelmengen sind nicht kritisch, aber unwirtschaftlich.

Man kann beispielsweise so vorgehen, dass man Base und Halogenalkylcarbonsäureanhydrid in einem Lösungsmittel vorlegt und 3-Amino-acrylsäurederivat zugibt.

In einer bevorzugten Ausführungsform des erfindungsgemäßen Verfahrens legt man 3-Amino-acrylsäurederivat und Base in einem Lösungsmittel vor und gibt das Halogenalkylcarbonsäureanhydrid zu.

Zur Aufarbeitung kann man beispielsweise so vorgehen, dass man gegebenenfalls ausgefallene Salze z.B. durch Filtration, Zentrifugation oder Sedimentation und Dekantieren abtrennt und die so erhaltene Reaktionslösung entweder direkt weiter umsetzt oder zur Gewinnung der 2-Halogenacyl-3-amino-acrylsäure-ester z.B. bis zur Trockene einengt.

Gegebenfalls können die 2-Halogenacyl-3-amino-acrylsäure-derivate durch Destillation weiter gereinigt werden, für den Einsatz zur Herstellung von 3-Halogenalkyl-4-pyrazolcarbonsäure-estern ist dies jedoch nicht nötig.

Auf erfindungsgemäße Weise werden beispielsweise und bevorzugt 2-Halogenacyl-3-amino-acrylsäure-ester der allgemeinen Formel (IV) erhalten, 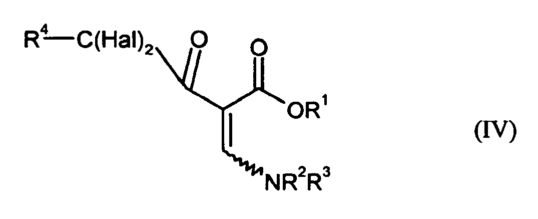 in der
- Hal und R⁴: jeweils unabhängig voneinander die gleiche Bedeutung und Vorzugs- bereiche besitzen, die unter der allgemeinen Formel (IIa) angegeben wurden und
- R² und R³: jeweils unabhängig voneinander die gleiche Bedeutung und Vorzugsbereiche besitzen, die unter der allgemeinen Formel (I) angegeben wurden.

Als bevorzugte Verbindungen der allgemeinen Formel (IV) seien genannt:
3-N,N-Dimethylamino-2-trifluoracetyl-acrylsäuremethylester,
3-N,N-Diethylamino-2-trifluoracetyl-acrylsäureethylester,
3-N,N-Dimethylamino-2-trichloracetyl-acrylsäuremethylester,
3-N,N-Diethylamino-2-trichloracetyl-acrylsäureethylester,
3-N,N-Dimethylamino-2-trichloracetyl-acrylsäureethylester,
3-N,N-Diethylamino-2-trichloracetyl-acrylsäuremethylester,
3-N,N-Dimethylamino-2-trifluoracetyl-acrylsäureethylester und
3-N,N-Diethylamino-2-trifluoracetyl-acrylsäureethylester.

Die auf erfindungsgemäße Weise hergestellten 2-Halogenacyl-3-amino-acrylsäurederivate eignen sich insbesondere zur Herstellung von 3-Halogenalkyl-4-pyrazol-carbonsäure-estern (Schritt b).
Beispielsweise und bevorzugt kann man die 2-Halogenacyl-3-amino-acrylsäure-ester der allgemeinen Formel (IV) durch Umsetzung mit Hydrazinen der allgemeinen Formel (V) gegebenenfalls in Gegenwart von Lösungsmittel in 3-Halogenalkyl-4-pyrazolcarbonsäure-ester der allgemeinen Formel (VI) überführen.

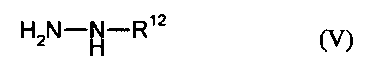

In der allgemeinen Formel (V) steht R¹² beispielsweise und bevorzugt für Wasserstoff, C₁-C₁₂-Alkyl, C₆-C₁₈-Aryl oder C₇-C₁₉-Arylalkyl, besonders bevorzugt für C₁-C₄-Alkyl.

Ganz besonders bevorzugt werden Hydrazin, Methylhydrazin und Ethylhydrazin eingesetzt, wobei Methylhydrazin noch weiter bevorzugt ist.

In der Formel (VI) 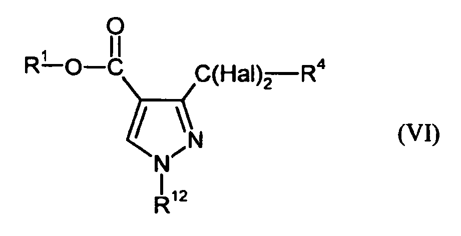 besitzen
- R¹: die gleiche Bedeutung und Vorzugsbereiche, die unter der allgemeinen Formel (I) angegeben wurden und
- Hal und R⁴: jeweils unabhängig voneinander die gleiche Bedeutung und Vor- zugsbereiche, die unter der allgemeinen Formel (IIa) angegeben wurden und
- R¹²: unabhängig davon die gleiche Bedeutung und Vorzugsbereiche besitzt, die unter der allgemeinen Formel (V) angegeben wurden.

Als bevorzugte Verbindungen der allgemeinen Formel (VI) seien genannt:
1-Methyl-3-trifluormethyl-4-pyrazolcarbonsäuremethylester
1-Methyl-3-trifluormethyl-4-pyrazolcarbonsäureethylester
1-Methyl-3-trichlormethyl-4-pyrazolcarbonsäuremethylester
1-Methyl-3-trichlormethyl-4-pyrazolcarbonsäureethylester

Bevorzugt wird die Umsetzung in Gegenwart von Lösungsmittel durchgeführt. Geeignete Lösungsmittel sind beispielsweise und bevorzugt diejenigen die oben für die Durchführung des Schritts a) angegeben wurden.

In einer besonders bevorzugten Ausführungsform des erfindungsgemäßen Verfahrens wird zur Herstellung der Verbindungen der allgemeinen Formel (VI), die gegebenenfalls nach Abtrennung von Feststoffen erhaltene Lösung aus Schritt a) verwendet.

Die Umsetzung mit Hydrazin kann beispielsweise und bevorzugt bei -30 bis +80°C, besonders bevorzugt bei -20 bis 25°C und ganz besonders bevorzugt bei -10 bis 10°C erfolgen.

Die 3-Halogenalkyl-4-pyrazolcarbonsäure-derivate können gegebenenfalls in an sich bekannter Weise (Houben-Weyl, Methoden der organischen Chemie, 4. Auflage, Band E5, S 223ff.) beispielsweise durch saure oder alkalische Verseifung in 3-Halogenalkyl-4-pyrazolcarbonsäuren der allgemeinen Formel (VII) überführt werden, 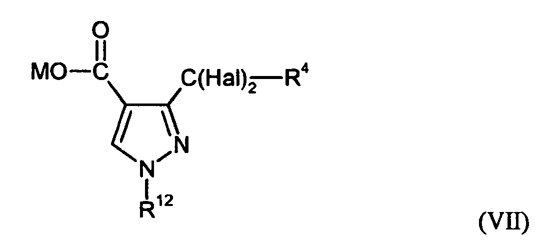 in der
- Hal und R⁴: jeweils unabhängig voneinander die gleiche Bedeutung und Vor- zugsbereiche besitzen, die unter der allgemeinen Formel (IIa) angegeben wurden und
- R¹²: unabhängig davon die gleiche Bedeutung und Vorzugsbereiche besitzt, die unter der allgemeinen Formel (V) angegeben wurden und M im Falle der alkalischen Verseifung für das Kation der eingesetzten Base oder nach ansäuern oder im Falle der sauren Verseifung für Wasserstoff steht.

Bevorzugt ist die alkalische Verseifung. Diese kann in an sich bekannter Weise beispielsweise durch Umsetzung mit Basen wie z.B. Alkalimetallhydroxiden wie z.B. Lithium-, Natrium- oder Kaliumhydroxid oder deren wässrige Lösungen erfolgen. Als Lösungsmittel sind beispielsweise Wasser, Alkohole wie z.B. Methanol, Ethanol und Isopropanol, aromatische Kohlenwasserstoffe wie z.B. Toluol, Aceton, Pyridin oder Mischungen solcher Lösungsmittel geeignet.

In einer bevorzugten Ausführungsform des erfindungsgemäßen Verfahrens wird zur Herstellung der Verbindungen der allgemeinen Formel (VII), die Reaktionslösung aus Schritt b) verwendet.

In einer besonders bevorzugten Ausführungsform des erfindungsgemäßen Verfahrens wird zur Herstellung der Verbindungen der allgemeinen Formel (VII) die Schritte a) b) und c) ohne Zwischenisolierung im gleichen Lösungsmittel, bevorzugt aromatischen Kohlenwasserstoffen wie z.B. Toluol durchgeführt.
Bevorzugte Verbindungen der allgemeinen Formel (VII) sind:
1-Methyl-3-trifluormethyl-4-pyrazolcarbonsäure, 3-Trifluormethyl-4-pyrazolcarbonsäure und 3-Trichlormethyl-4-pyrazolcarbonsäure.

Die erfindungsgemäß hergestellten 2-Halogenacyl-3-amino-acrylsäure-ester, 2-Halogenacyl-3-amino-acrylsäure-ester und Pyrazol-4-carbonsäuren bzw. deren Salze eignen sich insbesondere zur Anwendung in einem Verfahren zur Herstellung von Arzneimitteln und Agrochemikalien wie z.B. Fungiziden, Pestiziden und Herbiziden.

Das erfindungsgemäße Verfahren hat den Vorteil, dass man aus gut verfügbaren Substanzen 2-Halogenacyl-3-amino-acrylsäureester in Ausbeuten von über 95 % darstellen kann.

Ein weiterer Vorteil des erfindungsgemäße Verfahrens ist, dass man ohne Isolierung der 2-Halogenacyl-3-aminoacrylsäureester und ohne Wechsel des Lösungsmittels substituierte Pyrazol-4-carbonsäureester oder die Säuren gegebenenfalls in Form ihrer Salze gewinnen kann.

### Beispiele

### Beispiel 1

### Synthese von 3-N,N-Dimethylamino-2-trifluoracetyl-acrylsäureethylester (Methode I)

69 g N,N-Dimethylaminoacrylsäureethylester wurden in 87 g Toluol vorgelegt und bei -15°C mit 101 g Trifluoressigsäureanhydrid innerhalb von 1 Stunde versetzt. Anschließend wurde bei 25°C 1 Stunde nachgerührt, die Reaktionsmischung anschließend mit 87 g Toluol verdünnt und dann zur Reaktionsmischung 150 g Wasser zugesetzt. Es wurde nochmals 15 Minuten nachgerührt und die sich bildende organische Phase abgetrennt. Die wässrige Phase wurde mit 75 ml Toluol extrahiert, die organischen Phasen über Magnesiumsulfat getrocknet und das Lösungsmittel im Vakuum entfernt. Es wurden 100,01 g 3-N,N-Dimethylamino-2-trifluoracetyl-acrylsäureethylester mit in einer Reinheit von 98 % (GC) erhalten. Das entspricht einer Ausbeute von 85 % der Theorie.

Das Produkt kann auch als Lösung in Toluol ohne weitere Aufarbeitung für nachfolgende Synthesen weiter eingesetzt werden.

### Beispiel 2

### Synthese von 3-N,N-Dimethylamino-2-trifluoracetyl-acrylsäureethylester (Methode II)

128 g N,N-Dimethylaminoacrylsäureethylester, 98 g Triethylamin und 312 g Toluol wurden vorgelegt und bei 10°C 120 g Trifluoracetylchlorid innerhalb von 3,5 Stunden zugegeben. Anschließend wurde bei 10°C 1 Stunde nachgerührt, anschließend auf 50°C aufgeheizt und dann zur Reaktionsmischung 150 g Wasser zugesetzt. Es wurde nochmals 15 Minuten nachgerührt und die sich bildende organische Phase abgetrennt. Die wässrige Phase wurde mit Toluol extrahiert und die vereinigten organischen Phasen ohne weitere Aufarbeitung direkt für die Pyrazol-Bildung wieder eingesetzt. Es wurden 652 g toluolische Lösung mit einem Gehalt an 3-N,N-Dimethylamino-2-trifluoracetyl-acrylsäureethylester von 32 % (GC-ISTD) erhalten. Das entspricht einer Ausbeute von 98 % der Theorie.

### Beispiel 3

### Synthese von 3-N,N-Dimethylamino-2-trifluoracetyl-acrylsäureethylester (Methode III)

143 g N,N-Dimethylaminoacrylsäureethylester, 111 g Triethylamin und 182 g Toluol wurden vorgelegt und bei -10°C bis -5°C innerhalb von 3,5 Stunden 149 g Trifluoracetylchlorid zugegeben. Anschließend wurde die Suspension auf 50°C aufgeheizt, der Feststoff isoliert und mit Toluol nachgewaschen. Die kombinierten organischen Phasen wurden ohne weitere Aufarbeitung direkt für nachfolgende Synthesen eingesetzt. Es wurden 637 g toluolische Lösung mit einem Gehalt an 3-N,N-Dimethylamino-2-trifluoracetyl-acrylsäureethylester von 37 % (GC-ISTD) erhalten. Das entspricht einer Ausbeute von 98 % der Theorie.

### Beispiel 4

### Synthese von 1-Methyl-3-trifluormethyl-4-pyrazolcarbonsäureethylester

Zu 300 g der toluolischen Lösung aus Beispiel 2 wurde bei 0°C eine Lösung von 23 g Methylhydrazin gelöst in 87 g Toluol innerhalb von 90 Minuten zugetropft. Anschießend ließ man die Reaktionsmischung 1 Stunde bei 0°C nachrühren. Das Toluol wurde unter vermindertem Druck (<100 mbar) und Temperaturen von max. 45°C abdestilliert und währenddessen durch 100 g Wasser ersetzt. Die Suspension wurde zur Vervollständigung der Kristallisation in 45 Minuten auf 0°C abgekühlt, bei dieser Temperatur 15 Minuten nachgerührt und der Feststoff auf einer Fritte isoliert. Das Rohprodukt wurde mit 30 g n-Hexan gewaschen und bei RT im Vakuum getrocknet. Es wurden 73 g 1-Methyl-3-trifluormethyl-4-pyrazolcarbonsäureethylester erhalten. Dieses entspricht einer Ausbeute von 82 % der Theorie.

### Beispiel 5

### 1-Methyl-3-trifluormethyl-4-pyrazolcarbonsäure

50 g 1-Methyl-3-trifluormethyl-4-pyrazolcarbonsäureethylester wurden mit 177 g Toluol und 54 g 25 %iger Natronlauge vorgelegt und 15 Stunden unter Rückfluss gekocht. Die trübe Reaktionsmischung wurde auf 50°C abgekühlt und mit 69 g Wasser versetzt. Die wässrige Phase wurde bei 30°C durch Zugabe von 83 g 15 %iger Salzsäure auf einen pH-Wert von 1-2 gestellt, wobei das Produkt aus der Lösung ausfiel. Die Suspension wurde 30 Minuten bei Raumtemperatur und 30 Minuten bei 0°C nachgerührt und das Rohprodukt anschließend isoliert und 3 mal mit je 60 g kaltem (<10°C) Wasser gewaschen. Nach dem Trocknen bei Raumtemperatur und unter vermindertem Druck wurden 43 g 1-Methyl-3-trifluormethyl-4-pyrazolcarbonsäure erhalten. Das entspricht einer Ausbeute von 98 % der Theorie.

## Patentansprüche

1. Verfahren zur Herstellung von 2-Halogenacyl-3-amino-acrylsäure-estern, **dadurch gekennzeichnet, dass** N-substituierte 3-Amino-acrylsäureester mit Halogenalkylcarbonsäureanhydriden, wobei unter Halogenalkylcarbonsäureanhydride auch Halogenalkylcarbonsäurehalogenide als die gemischten Anhydride von Halogenalkylcarbonsäuren mit Halogenwasserstoffsäuren verstanden werden, in Gegenwart von Base umgesetzt werden.

2. Verfahren nach Anspruch 1, **dadurch gekennzeichnet, dass** es in Gegenwart von Lösungsmittel durchgeführt wird.

3. Verfahren nach einem oder mehreren der Ansprüche 1 bis 2, **dadurch gekennzeichnet, dass** als 3-Amino-acrylsäureester solche der allgemeinen Formel (I) eingesetzt werden, 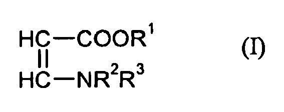 in der
R¹ für C₁-C₁₂-Alkyl, C₆-C₁₈-Aryl oder C₇-C₁₉-Arylalkyl steht und
R² und R³ jeweils unabhängig voneinander für C₁-C₁₂-Alkyl oder C₇-C₁₉- Arylalkyl stehen.

4. Verfahren nach einem oder mehreren der Ansprüche 1 bis 3, **dadurch gekennzeichnet, dass** als 3-Amino-acrylsäureester 3-(N,N-Dimethylamino)-acrylsäuremethylester eingesetzt wird.

5. Verfahren nach einem oder mehreren der Ansprüche 1 bis 4, **dadurch gekennzeichnet, dass** als Halogenalkylcarbonsäureanhydride solche der allgemeinen Formel (IIa) oder (IIb)eingesetzt werden, 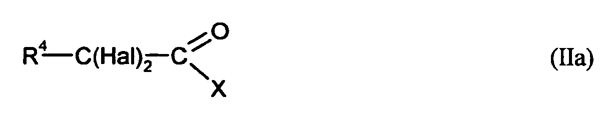 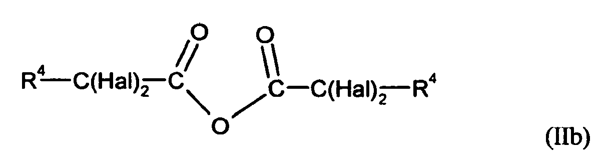 in der
X für Chlor, Brom oder Iod und
Hal jeweils unabhängig voneinander für Chlor oder Fluor und
R⁴ für Chlor, Fluor, C₁-C₁₂-Halogenalkyl, C₁-C₁₂-Alkyl, C₆-C₁₈-Aryl oder C₆-C₁₉-Arylalkyl steht.

6. Verfahren nach einem oder mehreren der Ansprüche 1 bis 5, **dadurch gekennzeichnet, dass** als Halogenalkylcarbonsäureanhydride Trifluoressigsäureanhydrid oder Trifluoressigsäurechlorid eingesetzt werden.

7. Verfahren nach einem oder mehreren der Ansprüche 1 bis 6, **dadurch gekennzeichnet, dass** als Basen tertiäre Stickstoffbasen eingesetzt werden.

8. Verfahren nach einem oder mehreren der Ansprüche 1 bis 7, **dadurch gekennzeichnet, dass** das molare Verhältnis von Base zu eingesetzten Halogenalkylcarbonsäureanhydriden 0,3 bis 3 beträgt.

9. Verfahren nach einem oder mehreren der Ansprüche 1 bis 8, **dadurch gekennzeichnet, dass** die Reaktionstemperatur -30 bis 120°C beträgt.

10. Verfahren nach einem oder mehreren der Ansprüche 1 bis 9, **dadurch gekennzeichnet, dass** das molare Verhältnis von Halogenalkylcarbonsäureanhydriden zu eingesetzten 3-Amino-acrylsäureestern 0,3 bis 1,5 beträgt.

11. Verfahren zur Herstellung von 3-Halogenalkyl-4-pyrazolcarbonsäure-ester **dadurch gekennzeichnet, dass**
a) N-substituierte 3-Amino-acrylsäureester mit Halogenalkylcarbonsäureanhydriden in Gegenwart von Base zu 2-Halogenacyl-3-amino-acrylsäure-estern umgesetzt werden und
b) die 2-Halogenacyl-3-amino-acrylsäure-ester mit Hydrazinen umgesetzt werden.

12. Verfahren nach Anspruch 11 **dadurch gekennzeichnet, dass** als Hydrazine solche der allgemeinen Formel (V) verwendet werden, 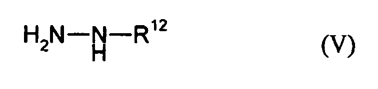 in der R¹² für Wasserstoff, C₁-C₁₂-Alkyl, C₆-C₁₈-Aryl oder C₇-C₁₉-Arylalkyl steht.

13. Verfahren nach einem oder mehreren der Ansprüche 11 bis 12, **dadurch gekennzeichnet, dass** Methylhydrazin verwendet wird.

14. Verfahren nach einem oder mehreren der Ansprüche 11 bis 13, **dadurch gekennzeichnet, dass** für Schritt b) eine gegebenenfalls von Feststoffen befreite Reaktionslösung aus Schritt a) verwendet wird.

15. Verfahren zur Herstellung von 3-Halogenalkyl-4-pyrazolcarbonsäuren oder deren Salzen **dadurch gekennzeichnet, dass**
a) 3-Amino-acrylsäureester mit Halogenalkylcarbonsäureanhydriden in Gegenwart von Base zu 2-Halogenacyl-3-amino-acrylsäure-estern umgesetzt werden und
b) die 2-Halogenacyl-3-amino-acrylsäure-ester mit Hydrazinen zu 3-Halogenalkyl-4-pyrazolcarbonsäureestern umgesetzt werden und
c) die Halogenalkyl-4-pyrazolcarbonsäureestern sauer oder alkalisch verseift werden.

16. Verfahren nach Anspruch 15 **dadurch gekennzeichnet, dass** die Halogenalkyl-4-pyrazolcarbonsäureester alkalisch verseift werden.

17. Verfahren nach einem oder mehreren der Ansprüche 15 bis 16 **dadurch gekennzeichnet, dass** die Schritte a), b) und c) im gleichen Lösungsmittel und ohne Zwischenisolierung von 2-Halogenacyl-3-amino-acrylsäure-estern oder Halogenalkyl-4-pyrazolcarbonsäureestern durchgeführt wird.

## Claims

1. Process for preparing 2-haloacyl-3-aminoacrylic esters, **characterized in that** N-substituted 3-aminoacrylic esters are reacted with haloalkylcarboxylic anhydrides, haloalkylcarboxylic anhydrides also being haloalkylcarbonyl halides as the mixed anhydrides of haloalkylcarboxylic acids with hydrohalic acids, in the presence of base.

2. Process according to Claim 1, **characterized in that** it is carried out in the presence of solvent.

3. Process according to one or more of Claims 1 and 2, **characterized in that** the 3-aminoacrylic esters used are of the general formula (I) 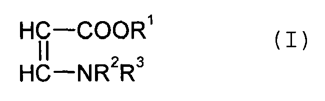 where
R¹ is C₁-C₁₂-alkyl, C₆-C₁₈-aryl or C₇-C₁₉-arylalkyl and
R² and R³ are each independently C₁-C₁₂-alkyl or C₇- C₁₉-arylalkyl.

4. Process according to one or more of Claims 1 to 3, **characterized in that** the 3-aminoacrylic ester used is methyl 3-(N,N-dimethylamino)acrylate.

5. Process according to one or more of Claims 1 to 4, **characterized in that** the haloalkylcarboxylic anhydrides used are of the general formula (IIa) or (IIb) 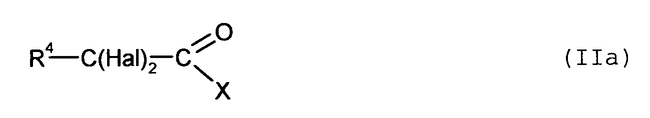 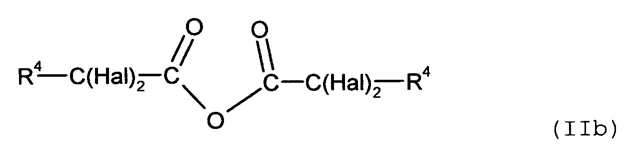 where
X is chlorine, bromine or iodine and
Hal are each independently chlorine or fluorine and
R⁴ is chlorine, fluorine, C₁-C₁₂-haloalkyl, C₁- C₁₂-alkyl, C₆-C₁₈-aryl or C₆-C₁₉-arylalkyl.

6. Process according to one or more of Claims 1 to 5, **characterized in that** the haloalkylcarboxylic anhydrides used are trifluoroacetic anhydride or trifluoroacetyl chloride.

7. Process according to one or more of Claims 1 to 6, **characterized in that** the bases used are tertiary nitrogen bases.

8. Process according to one or more of Claims 1 to 7, **characterized in that** the molar ratio of base to haloalkylcarboxylic anhydrides used is from 0.3 to 3.

9. Process according to one or more of Claims 1 to 8, **characterized in that** the reaction temperature is from -30 to 120°C.

10. Process according to one or more of Claims 1 to 9, **characterized in that** the molar ratio of haloalkylcarboxylic anhydrides to 3-aminoacrylic esters used is from 0.3 to 1.5.

11. Process for preparing 3-haloalkyl-4-pyrazolecarboxylic esters, **characterized in that**
a) N-substituted 3-aminoacrylic esters are reacted with haloalkylcarboxylic anhydrides in the presence of base to give 2-haloacyl-3-aminoacrylic esters and
b) the 2-halo-3-aminoacrylic esters are reacted with hydrazines.

12. Process according to Claim 11, **characterized in that** the hydrazines used are of the general formula (V) 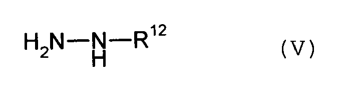 where R¹² is hydrogen, C₁-C₁₂-alkyl, C₆-C₁₈-aryl or C₇-C₁₉-arylalkyl.

13. Process according to one or more of Claims 11 and 12, **characterized in that** methylhydrazine is used.

14. Process according to one or more of Claims 11 to 13, **characterized in that** step b) is carried out using a reaction solution from step a) which has optionally been freed of solids.

15. Process for preparing 3-haloalkyl-4-pyrazolecarboxylic acids or salts thereof, **characterized in that**
a) 3-aminoacrylic esters are reacted with haloalkylcarboxylic anhydrides in the presence of base to give 2-haloacyl-3-aminoacrylic esters and
b) the 2-haloacyl-3-aminoacrylic esters are reacted with hydrazines to give 3-haloalkyl-4-pyrazolecarboxylic esters and
c) the haloalkyl-4-pyrazolecarboxylic esters are hydrolysed using acid or alkali.

16. Process according to Claim 15, **characterized in that** the haloalkyl-4-pyrazolecarboxylic esters are hydrolysed using alkali.

17. Process according to one or more of Claims 15 and 16, **characterized in that** steps a), b) and c) are carried out in the same solvent and without intermediate isolation of 2-haloacyl-3-aminoacrylic esters or haloalkyl-4-pyrazolecarboxylic esters.

## Revendications

1. Procédé pour la préparation d'esters de l'acide 2-halogénoacyl-3-aminoacrylique, **caractérisé en ce qu'**on transforme des esters d'acides 3-aminoacryliques N-substitués avec des anhydrides d'acides halogénoalkylcarboxyliques, où on entend par anhydrides d'acides halogénoalkylcarboxyliques également des halogénures d'acides halogénocarboxyliques ainsi que les anhydrides mixtes d'acides halogénoalkyliques avec des acides halogénohydriques, en présence d'une base.

2. Procédé selon la revendication 1, **caractérisé en ce qu'**il est réalisé en présence de solvants.

3. Procédé selon l'une ou plusieurs des revendications 1 à 2, **caractérisé en ce qu'**on utilise comme esters des acides 3-aminoacryliques ceux de formule générale (I), 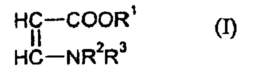 où
R¹ représente C₁-C₁₂-alkyle, C₆-C₁₈-aryle ou C₇-C₁₉- arylalkyle et
R² et R³ représentent, à chaque fois indépendamment l'un de l'autre, C₁-C₁₂-alkyle ou C₇-C₁₉-arylalkyle.

4. Procédé selon l'une ou plusieurs des revendications 1 à 3, **caractérisé en ce qu'**on utilise comme ester d'acide 3-aminoacrylique l'ester méthylique de l'acide 3-(N,N-diméthylamino)-acrylique.

5. Procédé selon l'une ou plusieurs des revendications 1 à 4, **caractérisé en ce qu'**on utilise comme anhydrides d'acides halogénoalkylcarboxyliques ceux de formule générale (IIa) ou (IIb), 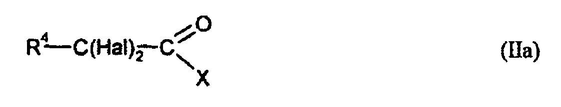 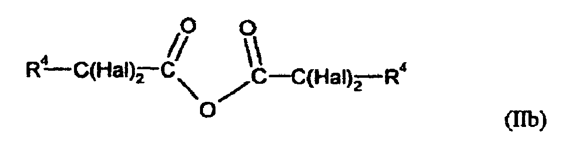 où
X représente chlore, brome ou iode et
Hal représente, à chaque fois, indépendamment l'un de l'autre, chlore ou fluor et
R⁴ représente chlore, fluor, C₁-C₁₂-halogénoalkyle, C₁- C₁₂-alkyle, C₆-C₁₈-aryle ou C₆-C₁₉-arylalkyle.

6. Procédé selon l'une ou plusieurs des revendications 1 à 5, **caractérisé en ce qu'**on utilise comme anhydrides d'acides halogénocarboxyliques l'anhydride de l'acide trifluoroacétique ou le chlorure de l'acide trifluoroacétique.

7. Procédé selon l'une ou plusieurs des revendications 1 à 6, **caractérisé en ce qu'**on utilise, comme bases des bases azotées tertiaires.

8. Procédé selon l'une ou plusieurs des revendications 1 à 7, **caractérisé en ce que** le rapport molaire de base à anhydrides d'acides halogénoalkylcarboxyliques utilisés est de 0,3 à 3.

9. Procédé selon l'une ou plusieurs des revendications 1 à 8, **caractérisé en ce que** la température de réaction est de -30°C à 120°C.

10. Procédé selon l'une ou plusieurs des revendications 1 à 9, **caractérisé en ce que** le rapport molaire d'anhydrides d'acides halogénoalkylcarboxyliques aux esters d'acides 3-aminoacryliques utilisé est de 0,3 à 1,5.

11. Procédé pour la préparation d'esters d'acides 3-halogénoalkyl-4-pyrazolecarboxyliques, **caractérisé en ce que**
a) des esters d'acides 3-aminoacryliques N-substitués sont transformés avec des anhydrides d'acides halogénoalkylcarboxyliques en présence d'une base en esters d'acides 2-halogénoacyl-3-aminoacryliques et
b) les esters d'acides 2-halogénoacyl-3-aminoacryliques sont transformés avec des hydrazines.

12. Procédé selon la revendication 11, **caractérisé en ce qu'**on utilise comme hydrazines celles de formule générale (V), 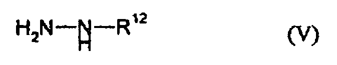 dans laquelle R¹² représente hydrogène, C₁-C₁₂-alkyle, C₆-C₁₈-aryle ou C₇-C₁₉-arylalkyle.

13. Procédé selon l'une ou plusieurs des revendications 11 à 12, **caractérisé en ce qu'**on utilise de la méthylhydrazine.

14. Procédé selon l'une ou plusieurs des revendications 11 à 13, **caractérisé en ce qu'**on utilise pour l'étape b) une solution réactionnelle de l'étape a), le cas échéant libérée des solides.

15. Procédé pour la préparation d'acides 3-halogénoalkyl-4-pyrazolecarboxyliques ou leurs sels, **caractérisé en ce que**
a) des esters d'acides 3-aminoacryliques sont transformés avec des anhydrides d'acides halogénoalkylcarboxyliques en présence d'une base en esters d'acides 2-halogénoacyl-3-aminoacryliques et
b) les esters d'acides 2-halogénoacyl-3-aminoacryliques sont transformés avec des hydrazines en esters d'acides 3-halogéno-4-pyrazolecarboxyliques et
c) les esters d'acides halogénoalkyl-4-pyrazolecarboxyliques sont saponifiés par voie acide ou alcaline.

16. Procédé selon la revendication 15, **caractérisé en ce que** les esters d'acides halogénoalkyl-4-pyrazolecarboxyliques sont saponifiés par voie alcaline.

17. Procédé selon l'une ou plusieurs des revendications 15 à 16, **caractérisé en ce que** les étapes a), b) et c) sont réalisées dans le même solvant et sans isolement intermédiaire des esters d'acides 2-halogénoacyl-3-aminoacryliques ou des esters d'acides halogénoalkyl-4-pyrazolecarboxyliques.
